# EUROPEAN PATENT APPLICATION

(11) **EP 2 295 013 A1**
(43) Date of publication of application: **16.03.2011**
(21) Application number: 09754795.4
(22) Date of filing: 29.05.2009
(51) Int. Cl.: A61F 13/15, A61F 13/49, A61F 13/496, A61F 13/511

(54) **ABSORBENT ARTICLE**

(30) Priority: 29.05.2008 JP 2008141544
(71) Applicant: Unicharm Corporation, Ehime 799-0111 (JP)
(72) Inventor: NAKAJIMA, Kaiyo, Kanonji-shi Kagawa 769-1602 (JP); MINATO, Hironao, Kanonji-shi Kagawa 769-1602 (JP); KAMIYAMA, Ryuichi, Kanonji-shi Kagawa 769-1602 (JP)
(74) Representative: Stark, Gordon Drummond
(86) International application number: PCT/JP2009/059842
(87) International publication number: WO 2009/145287

(57) **Abstract**

An absorbent article is improved to restrict a possibility that through-hole(s) formed in a separate sheet 3 might have width dimension(s) narrowed as the separate sheet is spaced from a liquid-absorbent structure 10. Outside of opposite lateral regions 30, 31 as viewed in a transverse direction X of the separate sheet, first front and rear elasticized regions 43, 44 are formed by front and rear waist elastic members 20, 21. Each of these first front and rear elasticized regions is paired so as to be spaced apart from each other in the transverse direction and between respective pairs of first front and rear elasticized regions, front and rear non-elasticized regions 45, 46 free from any influence by the contractile force of the front and rear waist elastic members. The sub-regions of the front and rear waist regions unoccupied by a liquid-absorbent structure 10 are provided with the front and rear waist elastic members attached thereto under tension so as to extend in the transverse direction between respective pairs of opposite side edges 14, 14 and 15, 15. Outside front and rear ends 27, 28 of the separate sheet 3 as viewed in a longitudinal direction Y, respectively, second front and rear elasticized regions 47, 48 are formed.

## Description

### TECHNICAL FIELD

The present invention relates to absorbent articles and particularly to absorbent articles such as disposable diapers, toilet training pants or incontinent briefs.

### RELATED ART

Disposable diapers provided above a topsheet thereof with a sheet formed with a through-hole for passage of feces and urine is known. For example, one of the diapers is disclosed in JP 2007-105299 A. According to the disclosure of JP 2007-105299 A, the diaper comprises a topsheet, a backsheet, an absorbent structure sandwiched between these topsheet and backsheet, and a skin-contact sheet provided above the topsheet. The skin-contact sheet is formed with a through-hole for passage of feces and a through-hole for passage of urine wherein a middle region is formed between these two through-holes. The skin-contact sheet is provided with through-hole biasing elastic members attached thereto under tension so that the skin-contact sheet may be spaced upward from the topsheet under a contractile force of these through-hole biasing elastic members and brought in contact with the wearer's skin. By keeping the skin-contact sheet in contact with the wearer's skin, the wearer's skin is protected from soil with body waste such as feces.
[PATENT DOCUMENT 1] JP 2007-105299 A

### DISCLOSURE OF THE INVENTION

### PROBLEM TO BE SOLVED BY THE INVENTION

The topsheet and the backsheet are provided in front and rear waist regions of the diaper with waist elastic members attached thereto under tension so as to extend in a transverse direction. The diaper is kept in close contact with the wearer's waist regions under contraction of these waist elastic members. However, this contraction of the waist elastic members may contract the skin-contact sheet so as to narrow the through-holes in the transverse direction. Particularly when the through-hole for passage of urine is narrowed, a peripheral edge of the through-hole may come in contact with the wearer's external genital and consequently cause skin troubles.

It is an object of the present invention to provide an absorbent article improved to reduce the possibility that through-hole (s) formed in a separate sheet corresponding to the aforesaid skin contactable sheet might have width dimension (s) narrowed as the separate sheet is spaced from a liquid-absorbent structure of an absorbent article.

### MEASURE TO SOLVE THE PROBLEM

According to the present invention, there is provided an improvement in an absorbent article comprising a chassis having a longitudinal direction and a transverse direction, sides facing the skin of a wearer and facing a garment of the wearer, a front waist region, a rear waist region and a crotch region extending between the front and rear waist regions, and a liquid-absorbent structure provided in the crotch region, and a separate sheet lying on the side facing the skin of the wearer and adapted to be spaced apart from the crotch region.

The improvement according to the present invention on a first aspect thereof is **characterized in that** the separate sheet comprises a pair of lateral regions extending in the longitudinal direction and opposed to each other in the transverse direction, a middle region defined between the lateral regions and being contiguous to the lateral regions, a through-hole defined in the side of the front waist region by the lateral regions and the middle region so as to communicate the side facing the skin of the wearer with the side facing the chassis, a pair of separate sheet elastic members attached under tension to the separate sheet so as to extend in the longitudinal direction and to be spaced apart from each other by the minimum distance in the middle region, and front and rear ends opposed to each other in the longitudinal direction and extending in the transverse direction and fixed to the side of the front and rear waist regions, respectively, the liquid-absorbent structure having front and rear ends opposed to each other in the longitudinal direction and extending in the transverse direction wherein the liquid-absorbent structure may be bowed so that the front and rear ends may be opposed to each other to form a void space between the separate sheet and the liquid-absorbent structure and to bring at least the middle region in contact with the skin of the wearer, and the front waist region is formed at least outside the lateral regions of the separate sheet as viewed in the transverse direction with a pair of first front elasticized regions adapted to be elastically contractible in the transverse direction wherein a front non-elasticized region is defined between the first front elasticized regions.

The improvement according to the present invention on a second aspect thereof is **characterized in that** the separate sheet comprises a pair of lateral regions extending in the longitudinal direction and opposed to each other in the transverse direction, a middle region defined between the lateral regions and being contiguous to the lateral regions, a through-hole defined in the side of the front waist region by the lateral regions and the middle region so as to communicate the side facing the skin of the wearer with the side facing the chassis, a pair of separate sheet elastic members attached under tension to the separate sheet so as to extend in the longitudinal direction and to be spaced apart from each other by the minimum distance in the middle region, and front and rear ends opposed to each other in the longitudinal direction and extending in the transverse direction and fixed to the side of the front and rear waist regions, respectively, the liquid-absorbent structure having front and rear ends opposed to each other in the longitudinal direction and extending in the transverse direction wherein the liquid-absorbent structure may be bowed so that the front and rear ends may be opposed to each other to form a void space between the separate sheet and the liquid-absorbent structure and to bring at least the middle region in contact with the skin of the wearer, and the front waist region is formed at least outside the lateral regions of the separate sheet as viewed in the transverse direction with a pair of first front elasticized regions adapted to be elastically contractible in the transverse direction wherein substantially no contractile force in the transverse direction acts on a region defined between the first front elasticized regions on the side of the liquid-absorbent structure facing the skin of the wearer.

According to one preferred embodiment, the rear waist region is formed outside the lateral regions of the separate sheet with a pair of first rear elasticized regions adapted to be elastically contractible in the transverse direction wherein a rear non-elasticized region is defined between the first rear elasticized regions.

According to another preferred embodiment, the middle region of the separate sheet lies in the crotch region, and the through-hole comprises a front through-hole broadening from the middle region toward the front end and a rear through-hole broadening from the middle region toward the rear end wherein the separate sheet elastic members extending on the lateral regions along the front and rear through-holes in the longitudinal direction.

According to still another preferred embodiment, the front non-elasticized region has a dimension corresponding to the maximum dimension of the front through-hole in the transverse direction and the rear non-elasticized region has a dimension corresponding to the maximum dimension of the rear through-hole in the transverse direction.

According to yet another preferred embodiment, the first front and rear elasticized regions have dimensions corresponding to length dimensions of the front and rear waist regions in the longitudinal direction, respectively.

According to further another preferred embodiment, the separate sheet elastic members extend to overlap the first front and rear elasticized regions in the front and rear waist regions.

According to an alternative preferred embodiment, the separate sheet is formed outside the front and rear ends as viewed in the longitudinal direction with second front and rear elasticized regions adapted to be elastically contractible in the transverse direction.

According to still further another embodiment, the lateral zones of the separate sheet are partially folded back onto and bonded to the skin side liner of the absorbent structure to define front and rear join zones.

### EFFECT OF THE INVENTION

The chassis is provided on its side facing the skin of the wearer with the separate sheet adapted to be spaced apart from the chassis and the separate sheet is provided with a pair of the separate sheet elastic members attached under tension thereto so as to extend in the longitudinal direction and to be spaced apart from each other by the minimum distance therebetween in the middle region. With such an arrangement, the middle regions is subjected to a contractile force in the transverse direction and a contractile force in the longitudinal direction so that this middle region is spaced apart upward from the chassis into contact with the skin of the wearer. The middle region kept in contact with the skin of the wearer ensures that urine and/or feces can be guided via the through-holes toward the liquid-absorbent structure and thereby to reduce body waste from clinging to the skin of the wearer.
The front waist region is formed outside the opposite lateral regions of the separate sheet as viewed in the transverse direction with the first front elasticized regions defined by the front waist elastic members so that the opposite lateral regions may be pulled outward in the transverse direction. In consequence, the width dimension of the through-hole defined between the opposite lateral regions are restricted from being narrowed and thereby potential skin troubles which would otherwise occur due to unacceptably narrowed width dimension of the through-hole can be substantially prevented. In addition, the front non-elasticized region or the region substantially free from any contractile force defined between a pair of the front elasticized regions ensures that no contractile force of the front waist elastic members is exerted upon the through-holes and thereby the shape of the through-hole is stabilized.

In the rear waist region, a pair of the first rear elasticized regions is defined outside the opposite lateral regions of the separate sheet as viewed in the transverse direction by the rear waist elastic members. In the rear waist region also, therefore, the width dimension of the through-hole is restricted from being narrowed, any potential skin troubles of the wearer can be thereby further reliably prevented and the shape of the through-hole can be stabilized.

The through-hole comprises the front through-hole gradually broadening from the middle region lying in the crotch region toward the front end and the rear through-hole gradually broadening from the middle region toward the rear end and the separate sheet elastic members are attached under tension to the separate sheet along these front and rear through-holes. With such an arrangement, the shape of the respective through-holes can be retained under the contractile force of the separate sheet elastic members. Consequently, any potential skin troubles of the wearer which would otherwise occur due to the narrowed through-holes can be prevented.

The front non-elasticized region has the width dimension corresponding to the maximum width dimension of the front through-hole in the transverse direction and the rear non-elasticized region has the width dimension corresponding to the maximum width dimension of the rear through-hole in the transverse direction. With such an arrangement, the opposite lateral regions extending outside the front and rear through-holes can be elastically pulled outward in the transverse direction without narrowing these front and rear through-holes in the transverse direction. Thus, the shapes of the front and rear through-holes can be retained and the width dimensions thereof can be prevented from being significantly narrowed.

The first front and rear elasticized regions respectively have length dimensions corresponding to the lengths of the front and rear waist regions, respectively, measured in the longitudinal direction. Such an arrangement ensures that the opposite lateral regions of the separate sheet can be pulled outward over the front and rear waist regions. In other words, the opposite lateral regions can be pulled outward over a wide range and thereby the dimensions of the through-holes in the transverse direction can be prevented further reliably from being narrowed.

In the front and rear waist regions, the separate sheet elastic members overlap the first front and rear elasticized regions so that not only the opposite lateral regions but also the separate sheet elastic members can be pulled outward in the transverse direction. In consequence, even if the separate sheet elastic members behave to reduce the dimensions of the respective through-holes in the transverse direction, such behavior can be effectively prevented.

Outside the front and rear ends of the separate sheet in the longitudinal direction, respectively, a pair of the second front elasticized regions and a pair of the rear elasticized regions are formed. In this way, the chassis is elastically contractible in the transverse direction fully around the wearer's waist so as to be maintained in close contact with the skin of the wearer and thereby to prevent bodily fluids such as urine from leaking out beyond the waist line.

### BRIEF DESCRIPTION OF THE DRAWINGS

Fig. 1 is a perspective view of a diaper according to a first embodiment of the inventive absorbent article.
Fig. 2 is a sectional view taken along the line II-II in Fig. 1.
Fig. 3 is a plan view of the diaper flatly developed.
Fig. 4 is an exploded perspective view of the diaper.
Fig. 5 is a plan view of a diaper flatly developed according to a second embodiment of the inventive absorbent article.
Fig. 6 is a sectional view taken along the line VI-VI in Fig. 5.

### IDENTIFICATION OF REFERENCE NUMERALS USED IN THE DRAWINGS

- 1: diaper (absorbent article)
- 2: chassis
- 3: separate sheet
- 7: front waist region
- 8: rear waist region
- 9: crotch region
- 10: liquid-absorbent structure
- 20: front waist elastic member
- 21: rear waist elastic member
- 30: lateral zone
- 31: lateral zone
- 32: middle zone
- 33: front through-hole
- 34: rear through-hole
- 39: separate sheet elastic member
- 43: first front elasticized region
- 44: first rear elasticized region
- 45: front non-elasticized region
- 46: rear non-elasticized region
- 47: second front elasticized region
- 48: second rear elasticized region
- 51: front flap
- 52: rear flap

### DETAILED DESCRIPTION OF THE PREFERRED EMBODIMENTS

Details of an absorbent article according to the present invention will be more fully understood from the description of a disposable diaper as an embodiment of the inventive absorbent article given hereunder with reference to the accompanying drawings.

### <First Embodiment>

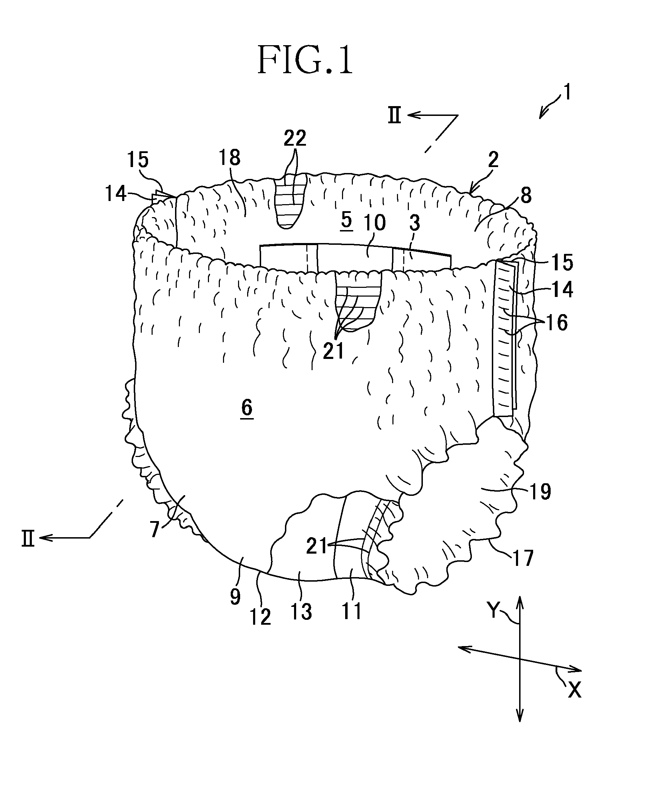

Figs. 1 through 4 illustrate a first embodiment of the present invention wherein Fig. 1 illustrates a diaper 1 with waist- and leg-openings opened. As illustrated, the diaper 1 according to this embodiment is of so-called pull-on type. The diaper 1 basically comprises a chassis 2 and a separate sheet 3. The chassis 2 is pant-shaped and comprises an inner side 5 facing the skin of a wearer, an outer side 6 facing a garment of the wearer, a front waist region 7, a rear waist region 8 and a crotch region 9 extending between said front and rear waist regions 7, 8. A direction extending from the front waist region 7 across the crotch region 9 to the rear waist region 8 is referred to herein as a longitudinal direction Y and a direction extending orthogonally to the longitudinal direction Y is referred to herein as a transverse direction X. The chassis 2 is constructed by an inner sheet 11, an outer sheet 12, a leak-barrier sheet 13 sandwiched between the inner sheet 11 and outer sheet 12, and a liquid-absorbent structure 10 disposed on the inner sheet 11.

The chassis 2 is shaped into a pull-on pant by putting the front waist region 7 and the rear waist region 8 flat and joined together along opposite side edges 14, 15 at a plurality of intermittently arranged joints 16.
Being joined together at the joints 16, a waist-opening 18 is defined by the front and rear waist regions 7, 8 as well as a pair of leg-openings 19 respectively are defined by the peripheral edges 17 of the crotch region 9. A plurality of waist elastic members 20, 21 extend along the peripheral edge of the waist-opening 18 and a plurality of leg elastic members 22 extend along the peripheral edges of the leg-openings 19, i.e., the peripheral edges 17 of the crotch region 9. The elastic members 20, 21, 22 are sandwiched between the inner sheet 11 and the outer sheet 12 and bonded under tension to at least one of the inner sheet 11 and the outer sheet 12 by adhesives (not shown).

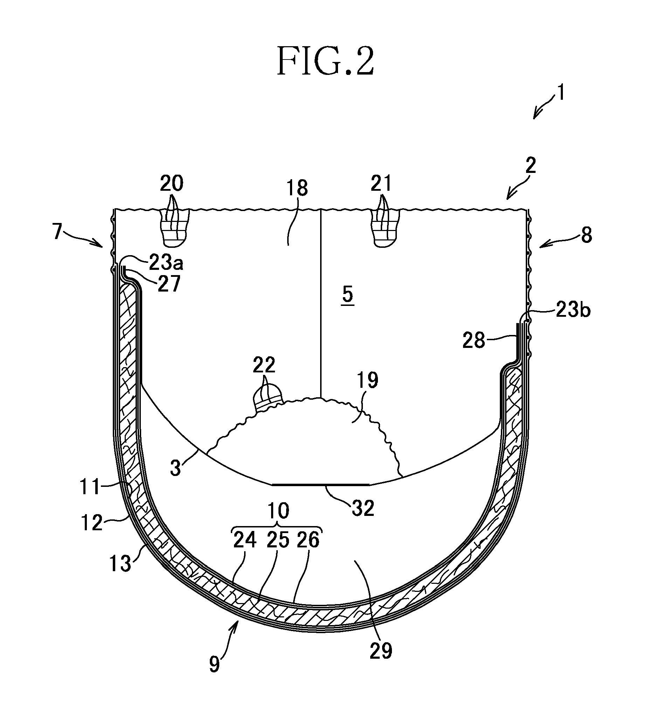

Fig. 2 is a sectional view taken along the line II-II in Fig. 1. As will be apparent from Fig. 2, the liquid-absorbent structure 10 is shaped into a panel 23 and provided on the inner side 5 of the inner sheet 11 and the separate sheet 3 is provided above the inner side of the liquid-absorbent structure 10. The liquid-absorbent structure 10 has front and rear ends 23a, 23b opposed to each other in the longitudinal direction and extending in the transverse direction.

The liquid-absorbent structure 10 extends in the longitudinal direction Y across the crotch region 9 into the front and rear waist regions 7, 8 and joined to the inner sheet 11 defining an inner surface of the front and rear waist regions 7, 8. The liquid-absorbent structure 10 comprises a liquid-absorbent core 25 wrapped with a liquid-absorbent/dispersant sheet 24 which is, in turn, covered with a skin side liner 26 facing the skin of the wearer. A side of the liquid-absorbent structure 10 facing a garment of the wearer is covered with the leak-barrier sheet 13 provided on the inner side of the outer sheet 12 so that bodily fluids once absorbed by the core 25 may be prevented from leaking out from the diaper 1. If it is desired, the leak-barrier sheet 13 may be bonded directly to the bottom surface of the liquid-absorbent structure 10. The inner sheet 11 and outer sheet 12 are formed, for example, of an air-permeable hydrophobic, fibrous nonwoven fabric and are bonded together by adhesives, or thermal or ultrasonic bonding means (not shown) by the intermediary of the leak-barrier sheet 13.

The separate sheet 3 is joined in the vicinity of its front and rear ends 27, 18 to the skin side liner 26 by adhesives, or thermal or ultrasonic bonding means (not shown). The front and rear ends 27, 28 respectively lie in the front and rear waist regions 7, 8. In a pull-on pant-shaped state as illustrated, the separate sheet 3 is spaced apart upward from the skin side liner 26 as a result of bowing of the crotch region 9 due to the contraction of separate sheet elastic members which will be described later. With the front and rear ends 27, 28 of the separate sheet 3 being joined to the skin side liner 26, the separate sheet 3 as a whole presents a shape like a hammock slung. Consequentially, a void space 29 is defined between the separate sheet 3 and the liquid-absorbent structure 10.

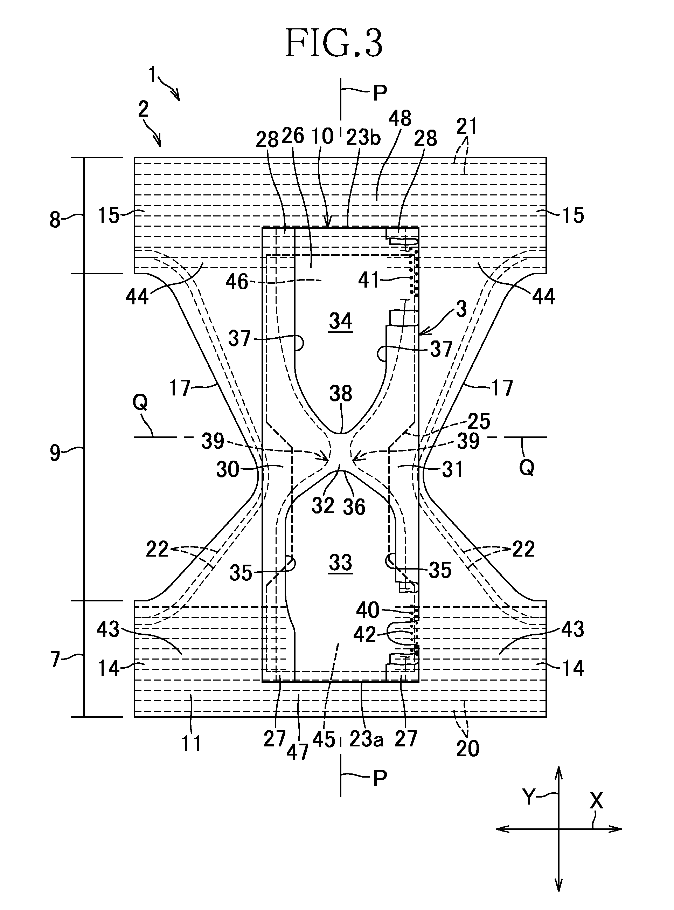

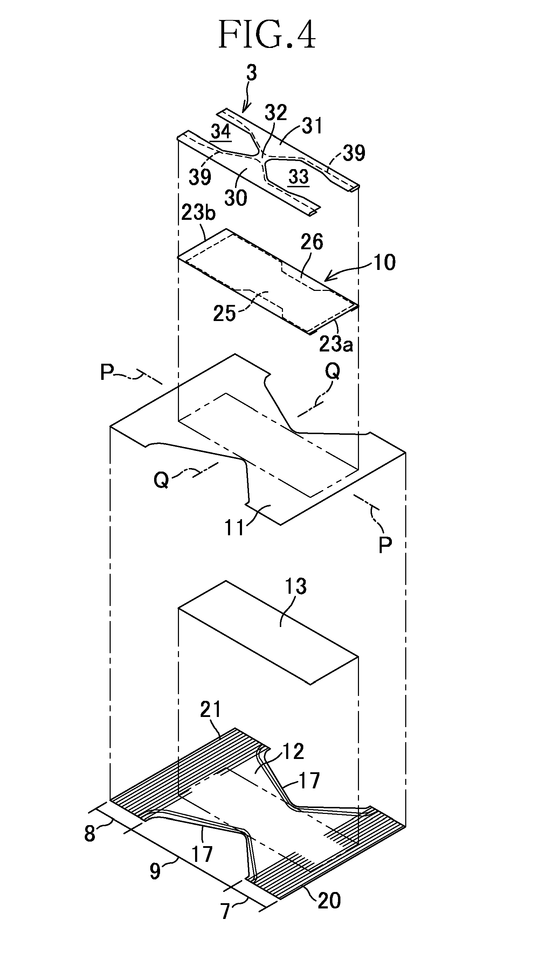

Fig. 3 is a plan view of the diaper 1 wherein the front and rear waist regions 7, 8 have been peeled off from each other at the joints 16 shown in Fig. 1 and then the diaper 1 has been developed and flattened in the longitudinal direction Y as well as in the transverse direction X so that the front and rear waist regions 7, 8 and the crotch region 9 may be coplanar, and Fig. 4 is an exploded perspective view corresponding to Fig. 3. In the state of the diaper 1 shown in Fig. 3, the elasticity of the respective elastic members provided in the diaper 1 is supposed to be inactive. The diaper 1 has a longitudinal center line P-P bisecting a dimension thereof in the transverse direction X and a transverse center line Q-Q bisecting a dimension thereof in the longitudinal direction Y. The diaper 1 is bilaterally symmetric about the longitudinal center line P-P.

The chassis 2 includes the transversely opposite side edges 14, 15 of the front and rear waist regions 7, 8 and transversely opposite leg-surrounding side edges 17, i.e., the peripheral edge 17 of the leg-openings 19. The leg-surrounding side edges 17 convexly curve toward the longitudinal center line P-P so that the chassis 2 as a whole is generally hourglass-shaped.
The front and rear waist elastic members 20, 21 and the leg elastic members 22 are sandwiched between the inner sheet 11 and the outer sheet 12. The front waist elastic member 20 comprises a plurality of elastic elements distributed in the front waist region 7 substantially at regular pitches in the longitudinal direction Y and attached to the chassis 2 under tension in the transverse direction X. The rear waist elastic member 21 also comprises a plurality of elastic elements distributed in the rear waist region 8 substantially at regular pitches in the longitudinal direction Y and attached to the chassis 2 under tension in the transverse direction X.

The liquid-absorbent structure 10 is laminated on the inner surface of the inner sheet 11 and the separate sheet 3 is laminated on the inner surface of the liquid-absorbent structure 10. The liquid-absorbent structure 10 and the separate sheet 3 have a substantially the same length direction in the longitudinal direction Y but smaller than a length dimension of the inner sheet 11 and the outer sheet 12 in the longitudinal direction Y. The liquid-absorbent core 25 constituting the liquid-absorbent structure 10 has a length dimension in the longitudinal direction Y smaller than that of the skin side liner 26 and wrapping the liquid-absorbent core 25. Consequentially, the front and rear ends 23a, 23b of the liquid-absorbent structure 10 are partially devoid of the liquid-absorbent core 25.

The separate sheet 3 comprises a pair of lateral zones 30, 31 opposed to each other in the transverse direction X and a middle zone 32 defined between and contiguous to the lateral zones 30, 31 wherein the middle zone 32 lies in the crotch region 9. The lateral zones 30, 31 and the middle zone 32 cooperate together to define front and rear through-holes 33, 34. The front through-hole 33 broadens from the middle zone 32 toward the front waist region 7 substantially in a U-shape and the rear through-hole 34 broadens from the middle zone 32 toward the rear waist region 8 substantially in a U-shape. The front through-hole 33 is defined by respective inner side edges 35 of the lateral zones 30, 31 and a curved margin of closure 36 making the inner side edges 35 contiguous to each other. The rear through-hole 34 is defined by respective inner side edges 37 of the lateral zones 30, 31 and a curved margin of closure 38 making the inner side edges 37 contiguous to each other.

The separate sheet 3 is provided with a pair of separate sheet elastic members 39 extending in the longitudinal direction Y along the front and rear through-holes 33, 34 and attached under tension to the separate sheet 3. The separate sheet elastic members 39 are attached to the separate sheet 3 in a substantially symmetrical relationship about the longitudinal center line P-P and are curved in the vicinity of the middle zone 32 toward the longitudinal center line P-P. The separate sheet 3 has the opposite lateral zones 30, 31 folded toward the side of the liquid-absorbent structure 10 in two-ply fashion. The separate sheet elastic members 39 are sandwiched between these two-ply layers of the lateral zones 30, 31 and bonded thereto by adhesives (not shown).

The separate sheet 3 extends across the crotch region 9 into the front and rear waist regions 7, 8 wherein the lateral zones 30, 31 are partially folded back onto and bonded to the skin side liner 26 of the liquid-absorbent structure 10 by adhesives (not shown) to define first front and rear joint zones 40, 41. The fold-back and bonded portions serve to reinforce the lateral zones 30, 31. These first front and rear joint zones 40, 41 are formed at least on the front and rear ends 27, 28 and preferably extend on the front and rear waist regions 7, 8 in the longitudinal direction Y but not on the crotch region 9. The liquid-absorbent structure 10 is joined to the inner sheet 11 by the intermediary of second joint zones 42. The second joint zones 42 fully extend across the front and rear waist regions 7, 8 and the crotch region 9 so as to overlap the first front and rear joint zones 40, 41 in the front and rear waist regions 7, 8.

The front and rear waist elastic members 20, 21 partially define first front and rear elasticized regions 43, 44 outside the lateral zones 30, 31 of the separate sheet 3 as viewed in the transverse direction X. Respective front ends 27 of the lateral zones 30, 31 extend into the front waist region 7 so that the front waist elastic members 20 are attached under tension so as to extend from one of the waist region's side edges 14 to the adjacent lateral zone 30, on one hand, and to extend from the other waist region's side edge 14 to the adjacent lateral zone 31, on the other hand. More specifically, the front waist elastic members 20 extend from the side edges 14 of the front waist region 7 across the first front joint zones 40 and the second joint zones 42 to the inner side edges 35 of the front through-hole 33. In like wise, respective rear ends 28 of the lateral zones 30, 31 extend into the rear waist region 8 so that the rear waist elastic members 21 are attached under tension so as to extend from one of the waist region's side edges 15 to the adjacent lateral zone 30, on one hand, and to extend from the other waist region's side edge 15 to the adjacent lateral zone 31, on the other hand. More specifically, the rear waist elastic members 21 extend from the side edges 15 of the rear waist region 8 across the first rear joint zones 41 and the second joint zones 42 to the inner side edges 37 of the front through-hole 34. The front and rear waist elastic members 20, 21 extending outward beyond the lateral zones 30, 31 in this manner define a pair of the first front elasticized regions 43 and a pair of the first rear elasticized regions 44 under contractile force of the front and rear waist elastic members 20, 21, respectively.

The first front elasticized regions 43 are spaced apart from each other in the transverse direction X to define therebetween a front non-elasticized region 45 substantially free from the contractile force of the front waist elastic members 20. The first rear elasticized regions 44 also are spaced apart from each other in the transverse direction X to define therebetween a rear non-elasticized region 46 substantially free from the contractile force of the rear waist elastic members 21. The front non-elasticized region 45 is dimensioned to have substantially the same length as the maximum width dimension of the front through-hole 33 as measured in the transverse direction X between the inner side edges 35 thereof so as to overlap the front through-hole 33 as exactly as possible. Similarly, the rear non-elasticized region 46 is dimensioned to have substantially the same length as the maximum width dimension of the rear through-hole 34 as measured in the transverse direction X between the inner side edges 37 thereof so as to overlap the rear through-hole 34 as exactly as possible. With such a unique arrangement, even if the front and rear through-holes 33, 34 are shaped so that the respective distances between the inner side edges 35, 35 and/or 37, 37 taper toward the front and rear ends 27, 28, respectively, the front and rear non-elasticized regions 45, 46 can keep the lengths corresponding to the maximum width dimensions between these inner side edges 35, 35 and/or 37, 37.

The sub-regions of the front and rear waist regions 7, 8 unoccupied by the liquid-absorbent structure 10 is provided with the front and rear waist elastic members 20, 21 continuously extending under tension in the transverse direction X between the side edges 14, 14 of the front waist region 7 and between side edges 15, 15 of the rear waist region 8, respectively. Thus second front and rear elasticized regions 47, 48 are defined outside the front and rear ends 27, 28 in the longitudinal direction Y, in other words, outside the front and rear ends 23a, 23b of the absorbent structure 10 in the longitudinal direction Y. It should be noted here that the front and rear waist elastic members 20, 21 partially overlap one another in the sub-region of the liquid-absorbent structure 10 unoccupied by the liquid-absorbent core 25. The front and rear ends 23a, 23b of the liquid-absorbent structure 10 may be partially provided with the front and rear waist elastic members 20, 21 in this manner to restrain the absorbent structure 10 from spaced apart from the skin of the wearer.

The first front and rear elasticized regions 43, 44 are spaced apart from each other in the transverse direction X in the front and rear waist regions 7, 8 and extend in the longitudinal direction Y along the side edges 14, 14, 15, 15 of the front and rear waist regions 7, 8, respectively. The second front and rear elasticized regions 47, 48 extend in the transverse direction X along the waist-opening and lie at least outside the front and rear ends 23a, 23b of the liquid-absorbent structure 10 as viewed in the longitudinal direction Y. According to this first embodiment, the second front and rear elasticized regions 47, 48 extend between the side edges 14 of the front waist region 7 and between the side edges 15 of the rear waist region 8, respectively, and may be in contact with or partially overlap the first front and rear elasticized regions 43, 44, respectively.

With the front and rear waist elastic members 20, 21 attached to the chassis 2, the first front and rear elasticized regions 43, 44, the front and rear non-elasticized regions 45, 46 and the second front and rear elasticized regions 47, 48 are formed by the following steps. The front and rear waist elastic members 20, 21 are attached under tension to at least one of the inner sheet 11 and the outer sheet 12 by adhesives so as to extend fully between the opposite side edges 14, 14 of the front waist region 7 and between the opposite side edges 15, 15 of the rear waist region 8 across not only the regions corresponding to the elasticized regions but also the regions corresponding to the non-elasticized regions. It should be noted here that the regions corresponding to the front and rear non-elasticized regions 45, 46 are not coated with adhesives. The front and rear waist elastic members 20, 21 are cut off or cut away in these regions left uncoated with adhesives to form the front and rear non-elasticized regions 43, 44 which are unaffected by contractile force of the front and rear waist elastic members 20, 21. The regions other than these front and rear non-elasticized regions 45, 46 define the first front and rear elasticized regions 43, 44 and the second front and rear elasticized regions 47, 48. In this way, the elasticized regions as well as the non-elasticized regions can be easily formed at a low cost merely by cutting off or cutting away the elastic members 20, 22 attached under tension.

With such diaper 1 put on the wearer's body, the separate sheet 3 is spaced apart upward to the skin of the wearer from the liquid-absorbent structure 10 under contractile force of the separate sheet elastic members 39 to define the void space 29 between the separate sheet 3 and the liquid-absorbent structure 10. The middle zone 32 of the separate sheet 3 lying in the crotch region 9 comes in contact with the crotch region of the wearer as the separate sheet 3 is spaced apart upward. In consequence, the external of the wearer genital of the wearer is aligned with the front through-hole 33 so that the wearer can urinate via the front through-hole into the void space 29 while the anus of the wearer is aligned with the rear through-hole 34 so that the wearer can defecate via the rear through-hole into the void space 29. In this way, liquid content of body waste is absorbed by the liquid-absorbent structure 10 and solid content thereof such as feces is prevented by the separate sheet 3 from clinging to the skin of the wearer. Consequentially, skin troubles due to such feces clinging to the skin can be effectively alleviated.

With the diaper 1 put on the wearer's body, the contractile force of the first front and rear elasticized regions 43, 44 functions to pull the liquid-absorbent structure 10 joined to the inner sheet 11 in the transverse direction X by the intermediary of the second joint zones 42. At the same time, the lateral zones 30, 31 of the separate sheet 3 joined to the liquid-absorbent structure 10 by the first front and rear joint zones 40, 41 are pulled outward in the transverse direction X. The contractile force of the front and rear waist elastic members 20, 21 are not exerted upon the front and rear non-elasticized regions 45, 46 defined between the first front and rear elasticized regions 43, 44 and therefore these front and rear non-elasticized regions 45, 46 are not contracted in the transverse direction X. Specifically, the front and rear through-holes 33, 34 are able to have stabilized shapes since there is no anxiety that respective width dimensions of these through-holes 33, 34 in the front and rear non-elasticized regions 45, 46 might be narrowed. Being ensured to have such stabilized width dimensions, potential skin troubles of the external genital of the wearer can be effectively prevented so far as the front through-hole 33 is concerned. As for the rear through-hole 34, feces can be reliably guided into the void space 29 and thereby the side of the separate sheet 3 facing the skin of the wearer can be reliably protected from being soiled with feces. This leads also to effective prevention of skin troubles.

In the front and rear non-elasticized regions 45, 46, the contractile force in the transverse direction X is not exerted also upon the liquid-absorbent structure 10. Consequentially, a potential formation of wrinkles can be restricted. Thereby leakage of body waste such as urine which might potentially occur should the liquid-absorbent structure 10 is formed with wrinkles. The front and rear waist elastic members 20, 21 overlap the regions of the liquid-absorbent structure 10 in which the liquid-absorbent core 25 is not present so as to form the second front and rear elasticized regions 47, 48. The second front and rear elasticized regions 47, 48 ensure that the liquid-absorbent structure 10 can be held in close contact with the skin of the wearer while the liquid-absorbent core 25 having a relatively high rigidity and adapted to become bulky as the core 25 absorbs urine is protected against formation of wrinkles.

While the front and rear non-elasticized regions 45, 46 have the same width dimensions as those of the front and rear through-holes 33, 34 in the transverse direction X according to the first embodiment, the present invention is not limited to such dimensioning. The front and rear non-elasticized regions 45, 46 as well as the first front and rear elasticized regions 43, 44 may be appropriately dimensioned so far as the first front and rear elasticized regions 43, 44 lie at least outside the lateral zones 30, 31 as viewed in the transverse direction X so that the lateral zones 30, 31 may be pulled by these first front and rear elasticized regions 43, 44. According to this embodiment, the front and rear non-elasticized regions 45, 46 are dimensioned to be about 40 mm or longer in the transverse direction X.

While the case in which the contractile force of the front and rear waist elastic members 20, 21 are not exerted upon the front and rear non-elasticized has been described above, the other cases may be contemplated in which such contractile force is substantially not exerted upon the side of the liquid-absorbent structure 10 facing the skin of the wearer. The expression "contractile force is substantially not exerted upon" used herein refers to, for example, the case in which the rigidity of the liquid-absorbent structure 10 substantially prevents the contractile force of the waist elastic members from acting upon the side of the liquid-absorbent structure 10 facing the skin of the wearer or makes it difficult for such contractile force to act upon the side of the liquid-absorbent structure 10 even when the waist elastic members are present in the front and rear non-elasticized regions 45, 46, or the case in which the segments of the waist elastic members extending in these non-elasticized regions have been cut off or cut away and consequently are devoid of the contractile force.
While the first and second elasticized regions 43, 44 are continuously formed in the longitudinal direction Y of the side edges 14, 15 of the front and rear waist regions 7, 8, respectively, in the illustrated embodiment, the present invention is not limited to such embodiment. For example, these front and rear elasticized regions 43, 44 may be formed only in sub-regions of the respective waist regions 7, 8 put aside toward the waist-opening or toward the crotch region 9. It is also possible to form these first front and rear elasticized regions 43, 44 discontinuously in the longitudinal direction Y.

Outside the front and rear ends 27, 28 of the separate sheet 3, the second front and rear elasticized regions 47, 48 are formed by the front and rear waist elastic members 20, 21 and thereby it is ensured that the diaper 1 can be maintained in close contact with the skin of the wearer and potential leakage of body waste such as urine beyond the waist-opening 18.

The separate sheet 3 is provided with a pair of separate sheet elastic members 39 curving inward in the middle region 32 of the separate sheet 3 so that a distance between the opposed elastic members 39 is minimized in this region 32. As a result, the contractile force of the separate sheet elastic members 39 act upon the middle region 32 in the transverse direction X as well as in the longitudinal direction Y so that the separate sheet 3 may be significantly spaced apart upward from the liquid-absorbent structure 10 toward the skin of the wearer until the middle region 32 may come in contact with the skin.

The separate sheet elastic members 39 having the minimum distance therebetween in the middle region 32 are spaced apart from each other in the transverse direction X as these elastic members 39 contract so as to pull the separate sheet 3 outward in the transverse direction X. Assumed that these separate sheet elastic members 39 extend in the longitudinal direction Y, intersect each other in the middle region 32 and extend again in the longitudinal direction Y, the separate sheet 3 can not be spaced apart from each other in the transverse direction X even when the separate sheet elastic members 39 contract. This is because, when one of these elastic members 39 contracts in the transverse direction X, the other contracts in the transverse direction X so as to follow the movement of the former. To avoid this, the separate sheet elastic members 39 are attached to the separate sheet 3 so as to be spaced apart even in the middle region 32. The distance between these separate sheet elastic members 39 is not specified so far as these elastic members 39 can contract so as to be spaced apart from each other in the transverse direction X. It should be appreciated that the distance by which the separate sheet elastic members 39 is preferably 2.7% - 11% of the length of the chassis 2 in the transverse direction X, for example, about 9.9 mm - 40 mm. If the distance is smaller than 2.7%, the front and rear through-holes 33, 34 will be unacceptably narrow in the transverse direction X and if the distance exceeds 11%, the distance by which the middle region 32 can be spaced apart from the liquid-absorbent structure 10 will be unacceptably short.

With the separate sheet elastic members 39 arranged to overlap the front and rear waist elastic members 20, 21, the separate sheet elastic members 39 also are pulled in the transverse direction X as the front and rear waist elastic members 20, 21 contract in the transverse direction X. The front and rear through-holes 33, 34 can be further restricted from being narrowed in the transverse direction X.
As stock materials for the front and rear waist elastic members 20, 21 as well as for the separate sheet elastic members 39, the illustrated embodiment employs rubber strands but the invention is not limited thereto and the other types of elastic member conventionally used in the relevant field of technique such as rubber belts may be used.

### <Second Embodiment>

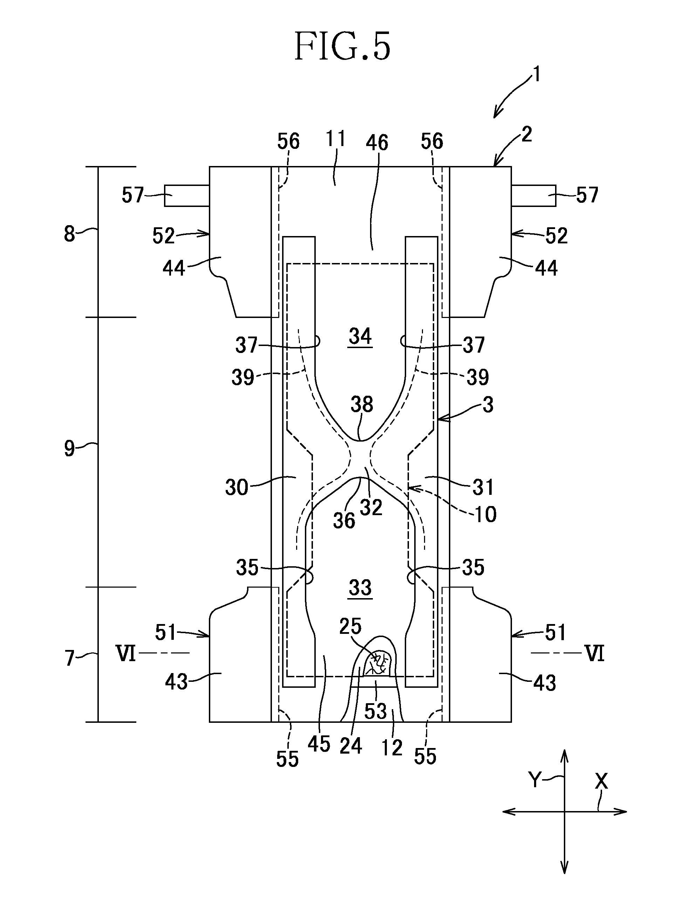

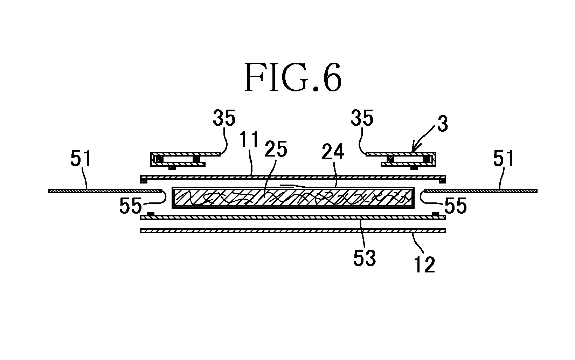

Fig. 5 is a plan view of a diaper 1 flatly developed according to the second embodiment and Fig. 6 is a sectional view taken along the line VI-VI in Fig. 5. The components of this second embodiment similar to those of the first embodiment are designated by the same reference numerals and detailed description of such components will be eliminated hereinafter. The diaper 1 according to the second embodiment comprises a chassis 2 and a separate sheet 3 wherein the chassis 2 comprises an inner sheet 11 and an outer sheet 12 which are substantially rectangular, a pair of front flaps 51 lying in a front waist region 7, a pair of rear flaps 52 lying in a rear waist region 8 and a liquid-absorbent structure 10 extending across a crotch region 9 into the front and rear waist regions 7, 8.

The liquid-absorbent structure 10 is sandwiched between the inner sheet 11 and the outer sheet 12 and shaped into a panel 23 as is the case with the first embodiment. The liquid-absorbent structure 10 comprises a liquid-absorbent core 25, a liquid-absorbent and liquid-dispersant sheet 24 wrapping the liquid-absorbent core 25 and a leak-barrier sheet 53 lying on the side of the liquid-absorbent and liquid-dispersant sheet 24 facing a wearer's garment. The outer sheet 12 is joined to the side of the leak-barrier sheet 53 facing the wearer's garment and the separate sheet 3 is attached directly to the side of the inner sheet 11 facing the wearer's skin.

Respective inner sides 55, 56 of front and rear flaps 51, 52 are sandwiched and attached between the inner sheet 11 and the outer sheet 12 so that these front and rear flaps 51, 52 extend outward in the transverse direction X. The front and rear flaps 51, 52 are formed of elastic sheets and attached to the inner sheet 11 and the outer sheet 12 so that these flaps 51, 52 are elastically contractible at least in the transverse direction X. The rear flaps 52 are provided on respective sides facing the garment of a wearer with fastening members 57 so that these fastening members 57 may be engaged with the sides of the associated front flaps 51 to put the diaper 1 on the wearer. These front and rear flaps 51, 52 define the first front and rear elasticized regions 43, 44, respectively.

While the respective inner side edges 55, 56 of these front and rear flaps 51, 52 are spaced apart from the lateral regions 30, 31 of the separate sheet 3 so that the front and rear flaps 51, 52 do not overlap the separate sheet 3 according to this second embodiment, the present invention is not limited to such an arrangement and these components may overlap one another.
Between a pair of first front elasticized regions 43 spaced apart from each other in the transverse direction X, a front non-elasticized region 45 upon which a contractile force of the front flaps 51 does not act is defined and, between a pair of first rear elasticized regions 44, a rear non-elasticized region 46 upon which a contractile force of the rear flaps 52 does not act is defined. In other words, the front and rear non-elasticized regions 45, 46 are not occupied by the front and rear flaps 51, 52, respectively, and accordingly free from the contractile force of these front and rear flaps 51, 52. The unique arrangement such that the respective inner side edges 55, 56 of the front and rear flaps 51, 52 do not overlap the separate sheet 3 allows the distance between a pair of the front flaps 51 as well as the distance between a pair of the rear flaps 52 to be widened and the length dimensions of the front and rear non-elasticized regions 45, 46 in the transverse direction X to be enlarged.

In the opposite lateral regions 30, 31, a pair of separate sheet elastic members 39 extends in the longitudinal direction Y along front and rear through-holes 33, 34 and curves inward in a middle region 32 so as to be spaced apart from each other by the minimum distance. Purpose of the separate sheet elastic members 39 is to space apart the separate sheet 3 from the liquid-absorbent structure 10 toward the wearer and therefore it is not essential to prolong front and rear ends thereof so as to overlap the front and rear waist regions 7, 8.

When it is intended to put such diaper 1 on the wearer, the rear flaps 52 may be stretched with the fastening members 57 held by the fingers of the wearer and these fastening members 57 may be fastened to the respective front flaps 52. Thereupon, both the front and rear flaps 51, 52 are subjected to a contractile force and function as the first front and rear elasticized regions 43, 44. The opposite lateral regions 30, 31 are pulled by the first front and rear elasticized regions 43, 44 outward in the transverse direction X so as to prevent the respective width dimensions of the front and rear through-holes in the transverse direction X from being narrowed. In consequence, potential skin troubles of the wearer can be effectively prevented.
In this manner, the front and rear non-elasticized regions 45, 46 can be formed by providing such front and rear flaps 51, 52 without any separate members, operations and any additional cost.

It is possible to provide the side of the separate sheet 3 with a pair of leak-barrier cuffs without departing from the scope of the invention. The leak-barrier cuff may be of the conventional type. The leak-barrier cuffs additionally provided in this manner not only can serve to prevent leakage of urine or the other liquid but also can serve to prevent the front and rear through-holes 33, 34 from being narrowed and to retain the desirable shapes of these through-holes 33, 34.

While a pair of the separate sheet elastic members 39 is curved inward in the middle region 32 so as to be spaced apart from each other by the minimum distance in the illustrated embodiment, it is not essential for these elastic members 39 to be curved so far as these elastic members 39 are spaced apart from each other by the minimum distance. For example, it is possible to attach these elastic members 39 so as to extend rectilinearly. In this case, these separate sheet elastic members 39 may be obliquely attached to the separate sheet 39 so that the distance between a pair of the separate sheet elastic members 39 is gradually broadened from the middle region 32 toward the front and rear waist regions 7, 8, respectively.

While each of the front and rear waist regions 7, 8 is formed with the elasticized region and the non-elasticized region in the illustrated embodiment, it is essential to form these regions at least in the front waist region 7. This is because, to prevent potential skin troubles due to contact of the front through-hole 33 with the external genital of the wearer, it is necessary to prevent the width dimension of the front through-hole 33 in the transverse direction X from being narrowed. The rear waist region 8 also may be formed with the elasticized region and the non-elasticized region to pull the separate sheet 3 as a whole outward in the transverse direction X and thereby to prevent the wearer's skin troubles further effectively.

While the separate sheet 3 is formed with the front and rear waist regions 7, 8 in the case of the illustrated embodiment, at least the front waist region 7 may be formed with the through-hole to meet the desired effect of the invention. However, the rear waist region 8 also may be formed with the through-hole to guide separately urine and feces toward the liquid-absorbent structure 10 via these two through-holes 33, 34. It is also possible to attach a sheet adapted to partition the void space 29 back and forth to the side of the middle region facing the liquid-absorbent panel 23. Such partitioning sheet makes it possible to minimize the possibility that urine and feces discharged into the void space might be mixed together.

The separate sheet 3 may be formed, for example, of a liquid-resistant and air-permeable nonwoven fabric, the inner sheet 11 and the outer sheet 12 may be formed, for example, of an air-permeable nonwoven fabric, the leak-barrier sheet 13 may be formed, for example, of a moisture-pervious plastic film, and the liquid-absorbent structure 10 may be formed, for example, from mixture of fluff pulp and super-absorbent polymer particles. All these stock materials have conventionally used in the relevant field of technique.

While the first embodiment has exemplarily described on the basis of the pull-on-type diaper and the second embodiment has exemplarily described on the basis of the open-type diaper, the diaper according to the first embodiment having the front and rear waist regions elasticized by the waist elastic members is applicable also to the open-type diaper and the diaper according to the second embodiment having the front and rear waist regions elasticized by the front and rear flaps is applicable also to the pull-on pants-type diaper.

## Claims

1. An absorbent article comprising a chassis having a longitudinal direction and a transverse direction, sides facing skin of a wearer and facing a garment of the wearer, a front waist region, a rear waist region and a crotch region extending between said front and rear waist regions, and a liquid-absorbent structure provided in said crotch region, and a separate sheet lying on said side facing the skin of the wearer and adapted to be spaced apart from said crotch region, said absorbent article being **characterized in that**:
said separate sheet comprises a pair of lateral regions extending in said longitudinal direction and opposed to each other in said transverse direction, a middle region defined between said lateral regions and being contiguous to said lateral regions, a through-hole defined in said front waist region by said lateral regions and said middle region so as to communicate said side facing the skin of the wearer with said side facing the chassis, a pair of separate sheet elastic members attached under tension to said separate sheet so as to extend in said longitudinal direction and to be spaced apart from each other by the minimum distance in said middle region, and front and rear ends opposed to each other in said longitudinal direction and extending in said transverse direction and fixed to the side of said front and rear waist regions, respectively;
said liquid-absorbent structure having front and rear ends opposed to each other in said longitudinal direction and extending in said transverse direction wherein said liquid-absorbent structure may be bowed so that said front and rear ends may be opposed to each other to form a void space between said separate sheet and said liquid-absorbent structure and to bring at least said middle region in contact with the skin of the wearer; and
said front waist region is formed at least outside said lateral regions of said separate sheet as viewed in said transverse direction with a pair of first front elasticized regions adapted to be elastically contractible in said transverse direction wherein a front non-elasticized region is defined between said first front elasticized regions.

2. An absorbent article comprising a chassis having a longitudinal direction and a transverse direction, sides facing the skin of a wearer and facing a garment of the wearer, a front waist region, a rear waist region and a crotch region extending between said front and rear waist regions, and a liquid-absorbent structure provided in said crotch region, and a separate sheet lying on said side facing the skin of the wearer and adapted to be spaced apart from said crotch region,
said separate sheet comprises a pair of lateral regions extending in said longitudinal direction and opposed to each other in said transverse direction, a middle region defined between said lateral regions and being contiguous to said lateral regions, a through-hole defined in said front waist region by said lateral regions and said middle region so as to communicate said side facing the skin of the wearer skin with said side facing said chassis, a pair of separate sheet elastic members attached under tension to said separate sheet so as to extend in said longitudinal direction and to be spaced apart from each other by the minimum distance in said middle region, and front and rear ends opposed to each other in said longitudinal direction and extending in said transverse direction and fixed to the side of said front and rear waist regions, respectively;
said liquid-absorbent structure having front and rear ends opposed to each other in said longitudinal direction and extending in said transverse direction wherein said liquid-absorbent structure may be bowed so that said front and rear ends may be opposed to each other to form a void space between said separate sheet and said liquid-absorbent structure and to bring at least said middle region in contact with the skin of the wearer; and
said front waist region is formed at least outside said lateral regions of said separate sheet as viewed in said transverse direction with a pair of first front elasticized regions adapted to be elastically contractible in said transverse direction wherein substantially no contractile force in said transverse direction acts on a region defined between said first front elasticized regions on the side of said liquid-absorbent structure facing the skin of the wearer.

3. The absorbent article according to Claim 1 or 2, wherein said rear waist region is formed outside said lateral regions of said separate sheet with a pair of first rear elasticized regions adapted to be elastically contractible in said transverse direction wherein a rear non-elasticized region is defined between said first rear elasticized regions.

4. The absorbent article according to any one of Claims 1 through 3, wherein:
said middle region of said separate sheet lies in said crotch region; and
said through-hole comprises a front through-hole broadening from said middle region toward said front end and a rear through-hole broadening from said middle region toward said rear end wherein said separate sheet elastic members extending on said lateral regions along said front and rear through-holes in said longitudinal direction.

5. The absorbent article according to Claim 4, wherein said front non-elasticized region has a dimension corresponding to the maximum dimension of said front through-hole in said transverse direction and said rear non-elasticized region has a dimension corresponding to the maximum dimension of said rear through-hole in said transverse direction.

6. The absorbent article according to any one of Claims 1 through 5, wherein said first front and rear elasticized regions have dimensions corresponding to length dimensions of said front and rear waist regions in said longitudinal direction, respectively.

7. The absorbent article according to any one of Claims 1 through 6, wherein said separate sheet elastic members extend to overlap said first front and rear elasticized regions in said front and rear waist regions.

8. The absorbent article according to any one of Claims 1 through 7, wherein said separate sheet is formed outside said front and rear ends as viewed in said longitudinal direction with second front and rear elasticized regions adapted to be elastically contractible in said transverse direction.

9. The absorbent article according to any one of Claims 1 through 7, said lateral zones of said separate sheet are partially folded back onto and bonded to said skin side liner of said absorbent structure to define front and rear join zones.
